(19) Europäisches Patentamt
European Patent Office
Office européen des brevets

(11) Veröffentlichungsnummer: **0 013 537**
**A2**

(12) # EUROPÄISCHE PATENTANMELDUNG

(21) Anmeldenummer: **79810173.9**

(22) Anmeldetag: **03.12.79**

(51) Int. Cl.³: **C 07 F 7/22**
C 08 K 5/58, C 07 C 149/18

(30) Priorität: **08.12.78 CH 12555/78**

(43) Veröffentlichungstag der Anmeldung:
**23.07.80 Patentblatt 80/15**

(84) Benannte Vertragsstaaten:
**DE FR GB IT**

(71) Anmelder: **CIBA-GEIGY AG**
**Patentabteilung Postfach**
**CH-4002 Basel(CH)**

(72) Erfinder: **Michaelis, Klaus-Peter, Dr.**
**Am hangweg 8**
**D-6145 Lindenfels/Odenwald(DE)**

(72) Erfinder: **Müller, Horst, Dr.**
**Reichenberger Strasse 12**
**D-6149 Fürth/Odenwald(DE)**

(54) **Organozinnverbindungen, ihre Anwendung zur Stabilisierung von chlorhaltigen Polymeren und die sie enthaltenden chlorhaltigen Polymere; Monothioglycerindiester.**

(57) Organozinnverbindungen der Formel

$$(C_1\text{-}C_{12}\text{-Alkyl})_n\text{-Sn-}(S\text{-}CH_2\underset{\underset{OR}{|}}{C}HCH_2\text{-}O\underset{\underset{O}{\|}}{C}C_{8\text{-}10}H_{17\text{-}21})_{4\text{-}n}$$

worin n 1 oder 2 ist und R ein $C_1$-$C_{18}$-Acylrest ist als Stabilisatoren fur halogenhaltige Polymere.

Monothioglycerindiester der Formel

$$HS\text{-}CH_2\text{-}\underset{\underset{OR}{|}}{C}H\text{-}CH_2\text{-}O\text{-}\underset{\underset{O}{\|}}{C}\text{-}C_{8\text{-}10}H_{17\text{-}21}$$

EP 0 013 537 A2

0013537



3-12149/CGM 205/+

C I B A - G E I G Y AG, BASEL

Neue Organozinnverbindungen

Die Erfindung betrifft neue Organozinnverbindungen, ihre Herstellung, ihre Verwendung als Stabilisatoren für halogenhaltige Polymere, sowie die so stabilisierten Polymere.

Aus der DE-OS 2.531.308 sind Mono- bzw. Di-organozinnmercaptohydroxyalkyl-carbonsäureester-monosulfide bekannt, so aus dem Beispiel 10 das Monomethylzinn-tris-(monothioglycerindiacetat), die zur Stabilisierung von Polyvinylchlorid und anderen Halogen enthaltenden Polymeren verwendet werden können.

In der praktischen Anwendung sind diese bekannten Organozinn-Stabilisatoren jedoch nicht immer zufriedenstellend, insbesondere hinsichtlich Geruchsfreiheit, Verarbeitungseigenschaften bei erhöhter Temperatur, thermischer Beständigkeit sowie im Fall spezieller Verarbeitungen, wie etwa bei einer Rohrrezeptur.

Aufgabe der vorliegenden Erfindung war es, diese Nachteile des Standes der Technik zu vermeiden und Stabilisatoren zu schaffen, die diese Nachteile nicht oder nur in geringerem Ausmass aufweisen.

Demgemäss betrifft die Erfindung Organozinnverbindungen der Formel I

$$(C_1\text{-}C_{12}\text{-Alkyl})_n\text{-Sn-}(S\text{-}CH_2\underset{\displaystyle OR}{CH}CH_2\text{-}O\underset{\displaystyle \|\atop O}{C}C_{8-10}H_{17-21})_{4-n} \quad (I),$$

worin n 1 oder 2 ist und R ein $C_1$-$C_{18}$-Acylrest ist.

In den Verbindungen der Formel I kann n 1 oder 2 sein, sodass Mono- und Di-alkyl-zinn-Verbindungen gemeint sind, die auch als Mischungen vorliegen können. Bevorzugt ist n aber 1.

Der Rest $-C(=O)C_{8-10}H_{17-21}$ stellt ein Gemisch dar für $-C(=O)C_8H_{17}$ und $-C(=O)C_{10}H_{21}$, wie dies im Handel erhältlich ist, kann aber auch für die einzelnen Bedeutungen stehen.

Der Rest R ist als $C_1$-$C_{18}$-Acyl bevorzugt $C_1$-$C_{18}$-Alkanoyl, $C_7$-$C_{11}$-Aroyl oder auch $C_4$-$C_{18}$-Alkoxycarbonylalkanoyl, wie Benzoyl, tert.-Butylbenzoyl, ein ($C_1$-$C_8$-Alkyl)-maleinsäurehalbester, wie Methylmaleinsäurehalbester, oder vor allem Acetyl oder Propionyl. Ebenso bevorzugt ist Acyl R aber auch Acetoacetyl.

Die an das Zinn gebundenen Alkylreste sind bevorzugt geradkettig und insbesondere Methyl, n-Butyl oder n-Octyl.

Bevorzugt sind also Verbindungen der Formel I, worin n 1 oder 2 ist und R $C_1-C_{18}$-Alkanoyl oder Acetoacetyl ist.

Vor allem bevorzugt sind Verbindungen der Formel I, worin n 1 ist und R Acetyl, Propionyl oder Acetoacetyl ist.

Ganz besonders bevorzugt sind Verbindungen der Formel I, worin das an das Zinn gebundene Alkyl Methyl, n-Butyl oder n-Octyl ist, n 1 ist und R Acetyl, Propionyl oder Acetoacetyl ist, sowie die in den Beispielen genannten Verbindungen, sowie $n-C_8H_{17}Sn[SCH_2CH(OCOCH_3)CH_2-O-COC_{10}H_{21}]_3$ und $n-C_8H_{17}Sn[SCH_2CH(OCOCH_2COCH_3)CH_2-O-COC_{10}H_{21}]_3$.

Die Herstellung der Verbindungen der Formel I kann nach an sich bekannten Methoden erfolgen, so nach den in der DE-OS 2.531.308 beschriebenen Verfahren, z.B. aus einem Zinnhalogenid $(C_1-C_{12}\text{-Alkyl})_n-SnHal_{4-n}$, einem Monothioglycerindiester der Formel II

$$HS-CH_2\underset{OR}{\underset{|}{C}}HCH_2-O\underset{O}{\underset{\|}{C}}C_{8-10}H_{17-21} \qquad (II) \quad ,$$

worin R obige Bedeutung hat. Die Umsetzung erfolgt bevorzugt in einem Lösungsmittel, insbesondere einem zweiphasigen Lösungsmittel, wie Wasser/organisches Lösungsmittel, z.B. Wasser/Alkan, wie Wasser/Heptan, vorteilhaft in Gegenwart eines Puffers, wie Natriumbicarbonat, und bei leicht erhöhter Temperatur, wie bei 30-60° C. Vorteilhaft setzt man 1 Mol Zinntrihalogenid mit 3 Mol Ester der Formel II um, bzw. 1 Mol Zinndihalogenid mit 2 Mol Ester der Formel II um. Man kann aber auch das Zinnhalogenid ganz oder teilweise durch ein entsprechendes Zinnoxid ersetzen und je nach eingesetzter Menge davon sogenannte überbasische Zinnverbindungen erhalten, die ebenfalls Gegenstand der Erfindung sind.

Die Ester der Formel II sind neu und bilden ebenfalls einen Gegenstand der Erfindung. R hat darin die oben allgemein und bevorzugt angegebene Bedeutung und steht vor allem für Acetyl oder Propionyl. Sie sind geeignet, in obigem Verfahren zur Herstellung von Verbindungen der Formel I verwendet zu werden.

Die erfindungsgemässen Stabilisatoren eignen sich hervorragend zum Schutz gegen den Abbau durch Wärme- und Lichteinwirkung von chlorhaltigen Thermoplasten. Im allgemeinen werden dem Kunststoff Verbindungen der Formel I in Mengen von 0,01 bis 10, vorzugsweise 0,1 bis 5 Gew.-% einverleibt. Ein weiterer Gegenstand vorliegender Erfindung sind damit thermoplastische Formmassen, enthaltend 0,01 bis 10 Gew.-%, bezogen auf die thermoplastische Mischung eines Stabilisators der Formel I.

Als chlorhaltige Thermoplaste seien genannt: Polyvinylidenchlorid, nachchlorierte Polyolefine und bevorzugt Polymere aus oder auf der Grundlage von Vinylchlorid. Als solche kommen E-, S- und M-PVC in Frage, wobei das Polyvinylchlorid auch weichmacherhaltig und nachchloriert sein kann. Insbesondere ist jedoch Hart-PVC bevorzugt, aus dem z.B. Fertigteile für die Aussenanwendung nach bekannten Verfahren wie Spritzgiessen oder Extrudieren hergestellt werden können.

Als Comonomere für Thermoplaste auf der Grundlage von Vinylchlorid seien genannt: Vinylidenchlorid, Transdichloräthen, Aethylen, Propylen, Butylen, Malein-

BAD ORIGINAL

säure, Acrylsäure, Fumarsäure, Itaconsäure oder Vinylacetat.

Vor, während oder nach der Zugabe des erfindungsgemässen Stabilisators können je nach Verwendungszweck der Formmasse weitere Zusätze einverleibt werden wie weitere Stabilisatoren, Gleitmittel, bevorzugt Montanwachse oder Glycerinester, Füllstoffe, verstärkende Füllstoffe, wie Glasfasern,und Modifikatoren, wie etwa Schlagzähzusätze.

Die nachfolgenden Beispiele erläutern die Erfindung näher. Teile sind Gewichtsteile und Prozente Gewichtsprozente.

Beispiel 1

250 ml Methanol werden mit 2,5 g Natriumhydrogensulfid versetzt und die Lösung bei 25°C mit Schwefelwasserstoff gesättigt. Anschliessend tropft man innerhalb von 2 Stunden 228 g "CARDURA-E-10"-Epoxid

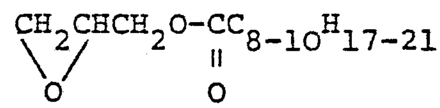

zu, wobei ständig Schwefelwasserstoff in der Menge eingeleitet wird, die das Reaktionsgemisch gerade aufnehmen kann. Die Temperatur darf 25 - 30°C nicht überschreiten, um die Bildung entsprechender Thioäther zu unterbinden.

Den Katalysator wäscht man mit wenig verd. Schwefelsäure aus, extrahiert mit Aether und entfernt das Lösungsmittel im Vakuum. Man erhält den Monothioglycerinester $HSCH_2CH\ (OH)CH_2\text{-}O\text{-}COC_{8-10}H_{17-21}$, der direkt weiter verwendet werden kann.

Beispiel 2

260 g des im Beispiel 1 beschriebenen Monothioglycerinesters verdünnt man mit 145 g Acetanhydrid. In diese Lösung tropft man unter Eiswasserkühlung 15 ml conc. Schwefelsäure. Die Temperatur sollte 40°C nicht überschreiten. Nach 1 Stunde Rühren verdünnt man mit Wasser, extrahiert mit Aether, wäscht mit gesättigter Natriumhydrogencarbonatlösung neutral und erhält den Mercaptodiester

$$HSCH_2CH(OCOCH_3)CH_2\text{-}O\text{-}COC_{8-10}H_{17-21}$$

als vollkommen geruchloses Oel nach Destillation bei 130°C/0,1 Torr mit einem SH-Gehalt von 10,2%.

Beispiel 3

260 g Mercaptoglycerinmonoester aus Beispiel 1 werden mit 130 g Propionsäureanhydrid versetzt und in

BAD ORIGINAL

diese Lösung unter Eiswasserkühlung 20 ml Conz. Schwefelsäure eingetropft. Die Temperatur sollte 40°C nicht übersteigen. Nach 1 Stunde Rühren verdünnt man mit Wasser, extrahiert mit Aether, wäscht mit ges. Natriumhydrogencarbonatlösung neutral und erhält den Mercaptodiester

$$HSCH_2CH(OCOCH_2CH_3)CH_2-O-COC_{8-10}H_{17-21}$$

als völlig geruchsfreies Oel, kp. SH-Gehalt 9,4%.

Beispiel 4

52,4 g Mercaptoglycerinmonoester aus Beispiel 1 werden mit 150 ml Acetessigsäureäthylester versetzt und solange erhitzt bis insgesamt 10 ml Aethanol abdestillieren. Nach Abkühlen entfernt man überschüssigen Acetessigsäureäthylester im Vakuum und erhält in quantitativer Ausbeute den acetoacetylierten Mercaptoglycerinester als farb- und geruchlose Verbindung mit einem SH-Gehalt von 9,3%.

Beispiel 5

Zu 700 g Mercaptodiester des Beispiels 2 mit einem Mercaptogehalt von 9,2% wurden 155,3 g Mono-n-butylzinnoxid (MBTO) mit einem Gesamtzinngehalt von 50,6% Sn und davon einem Monozinngehalt von 48,0% auf ca. 80°C unter Rühren erhitzt und das entstehende Reaktionswasser im Vakuum abgezogen. Nach Abkühlen wird das Reaktionsprodukt von verbliebenen Trübungen filtriert und man erhält 837 g Monobutylzinnmercaptid

$$n-C_4H_9Sn[SCH_2CH(OCOCH_3)CH_2-O-COC_{8-10}H_{17-21}]_3$$

als klares, geruchsfreies Produkt.

| | | |
|---|---|---|
| Analyse: | gefunden: | 9,35 % Gesamtzinn; 9,1 % Sn (I) |
| | berechnet: | 9,38 % Gesamtzinn; 8,9 % Sn (I) |

Beispiel 6

Zu 30 g Mercaptodiester des Beispiels 2 mit einem Mercaptogehalt von 9,2 % werden 10,3 g Di-n-butylzinnoxid (DBTO) hinzugefügt und unter Rühren auf ca. 80°C erwärmt. Das dabei entstehende Reaktionswasser wird im Vakuum entfernt. Das DBTO geht dabei bis auf wenige polymere Verunreinigungen in Lösung die durch Filtrieren entfernt werden. Es bleiben 39,5 g Dibutylzinndimercaptid

$$(n-C_4H_9)_2Sn[SCH_2CH(OCOCH_3)CH_2-O-COC_{8-10}H_{17-21}]_2$$

als klares, viskoses, völlig geruchsloses Produkt.

Analyse: gefunden: 12,60 % Sn (II)
berechnet: 12,55 % Sn (II)

Beispiel 7 (Vergleichsbeispiel)

$$n-C_4H_9Sn[SCH_2CH(-OCOCH_3)CH_2-OCOCH_3]_3$$

60,6 g Monobutylzinnoxid (Gehalt: 0,245 g Atom monoalkyliertes Zinn und 0,009 g Atom dialkyliertes Zinn) wurden mit 146,2 g $HSCH_2CH(OCOCH_3)CH_2OCOCH_3$ (0,761 Mol) bei 110° 1 h lang erhitzt und das Reaktionswasser im Wasserstrahlvakuum abgezogen. Es resultierte eine farblose, zähe Flüssigkeit.

Beispiel 8

Eine zur Herstellung von Hart-PVC-Rohren geeignete Rezeptur hatte folgenden Aufbau:

| | |
|---|---|
| S-PVC (K-Wert 68) | 100,0 Teile |
| Kreide | 1,0 " |
| Titandioxid | 1,0 " |
| Calcium-Stearat | 0,8 " |
| Paraffinwachs | 0,8 " |

Monoorganozinnmercaptid 0,5 - 0,8 "

Als erfindungsgemässes Monoorganozinnmercaptid wurde der in Beispiel 5 beschriebene Stabilisator mit 0,5 % (= 0,046% Zinn) und 0,8% (=0,074% Zinn) und als Vergleichsstabilisator der nach Beispiel 7 hergestellte Stabilisator mit 0,5 % (=0,077 % Zinn) eingesetzt. Nach intensiver Vermischung der Compounds wurden bei 200° Dauerwalzversuche durchgeführt und im zeitlichen Abstand von 3 min Proben entnommen, deren Verfärbung gemessen wurde (Yellowness Index). Man vergleiche Tabelle 1.

Tabelle 1

| Stab. \ Zeit (Minuten) | | 3' | 6' | 9' | 12' | 15' | 18' | 21' | 24' | 27' |
|---|---|---|---|---|---|---|---|---|---|---|
| Stab. aus Beispiel 5 | 0,5 % | 13,2 | 15,5 | 21,2 | 33,9 | 54,8 | | | | |
| | 0,8 % | 8,6 | 10,7 | 13,2 | 17,2 | 22,5 | 31,0 | 41,2 | 55,6 | 66,0 |
| Vergleichs-stab.aus Beispiel 7 | 0,5 % | 9,8 | 12,2 | 20,4 | 41,4 | 56,7 | | | | |

Tabelle 1 lehrt, dass bei Einsatzmengen auf gleicher Zinnbasis der erfindungsgemäss hergestellte Stabilisator dem Vergleichsstabilisator sowohl in der Anfangsfarbe als auch der Langzeitstabilität deutlich überlegen ist und selbst bei gewichtsgleichem Einsatz eine vergleichbare Langzeit-stabilität erzielt wird.

0013537



Beispiel 9

Eine Hart-Folien-Rezeptur hatte folgenden Aufbau:

| | |
|---|---|
| S-PVC (K-Wert 64) | 100,0 Teile |
| 1.3-Butandiolmontansäureester | 0,2 " |
| Glycerinmonooleat | 1,0 " |
| Organozinnstabilisator 0,6 - | 1,6 % |

Als Organozinnstabilisatoren fungierten:

1 % (= 0,092 % Zinn) bzw. 1,6 % (= 0,144 % Zinn) des erfindungsgemässen Stabilisators von Beispiel 5 und 0,6 % (= 0,092 % Zinn) bzw. 1,0 % (= 0,153 % Zinn) des in Beispiel 7 beschriebenen Vergleichsstabilisators.

Nach gründlicher Vermischung der Compounds wurde ein Dauerwalztest bei 190° durchgeführt und im zeitlichen Abstand von 5 min. Proben entnommen. Die in Abhängigkeit von der Belastungszeit erhaltenen Verfärbungen (Yellowness Indixes) sind in Tabelle 2 aufgeführt.

Tabelle 2

| Zeit (Min) / Stab. | 5' | 10' | 15' | 20' | 25' | 30' | 35' | 40' | 45' | 50' | 55' | 60' |
|---|---|---|---|---|---|---|---|---|---|---|---|---|
| Stab. aus Beisp. 5 1,0% | 4,0 | 5,6 | 9,2 | 12,8 | 25,2 | 29,9 | 50,1 | 84,3 | | | | |
| Stab. aus Beisp. 5 1,6 % | 3,5 | 4,4 | 7,3 | 10,7 | 20,1 | 24,7 | 31,3 | 34,1 | 38,3 | 50,9 | 77 | 96,0 |
| Vergleichsstab. Beisp. 7 0,6% | 4,6 | 10,5 | 27,9 | 53,9 | 75,7 | 85,1 | | | | | | |
| Vergleichsstab. Beisp. 7 1,0% | 4,0 | 6,5 | 11,9 | 29,4 | 49,2 | 65,7 | 79,3 | | | | | |

Aus Tabelle 2 geht hervor, dass der erfindungsgemässe Stabilisator bei zinn gleicher Einsatzmenge dem Vergleichsstabilisator eindeutig und sogar bei mengengleichem Einsatz noch deutlich überlegen ist.

Beispiel 10

$$n\text{-}C_4H_9Sn[SCH_2CH(OCOCH_2COCH_3)\ CH_2\text{-}O\text{-}COC_{8\text{-}10}H_{17\text{-}21}]_3$$

7,31 g Mono-n-butylzinnoxid (Gehalt: 0,0295 Grammatom monoalkyliertes Zinn und 0,01303 Grammatom dialkyliertes Zinn) wurden mit 33,70 g

$$HSCH_2CH(OCOCH_2COCH_3)\ CH_2\text{-}O\text{-}CO\ C_{8\text{-}10}H_{17\text{-}21}$$

(94,7%ig = 0,0922 Mol) bei 110° eine Stunde lang umgesetzt und das Reaktionswasser im Wasserstrahlvakuum entfernt. Es resultierte eine geruchsfreie viskose Flüssigkeit (Zinn berechnet: 9,2%; Zinn gefunden: 9,3%).

## Patentansprüche:

1. Organozinnverbindungen der Formel I

$$(C_1-C_{12}\text{-Alkyl})_n\text{-Sn-}(S\text{-}CH_2\underset{\substack{|\\ OR}}{C}HCH_2\text{-}O\underset{\substack{\|\\ O}}{C}C_{8-10}H_{17-21})_{4-n} \quad (I),$$

worin n 1 oder 2 ist und R ein $C_1$-$C_{18}$-Acylrest ist.

2. Verbindungen der Formel I nach Anspruch 1, worin n 1 oder 2 ist und R $C_1$-$C_{18}$ Alkanoyl oder Acetoacetyl ist.

3. Verbindungen der Formel I nach Anspruch 1, worin n 1 ist und R Acetyl, Propionyl oder Acetoacetyl ist.

4. Verbindungen der Formel I nach Anspruch 1, worin das an das Zinn gebundene Alkyl Methyl, n-Butyl oder n-Octyl ist, n 1 ist und R Acetyl, Propionyl oder Acetoacetyl.

5. Verbindung nach Anspruch 1, $n\text{-}C_8H_{17}Sn[SCH_2CH(OCOCH_3)CH_2\text{-}O\text{-}COC_{10}H_{21}]_3$.

6. Verwendung von Verbindungen der Formel I nach einem der Ansprüche 1-5 zur Stabilisierung von chlorhaltigen Polymeren.

7. Chlorhaltige Polymere, stabilisiert mit einer Verbindung nach einem der Ansprüche 1-5.

8. Monothioglycerindiester der Formel II

$$HS\text{-}CH_2\underset{\substack{|\\ OR}}{C}HCH_2\text{-}O\underset{\substack{\|\\ O}}{C}C_{8-10}H_{17-21} \quad (II),$$

worin R ein $C_1$-$C_{18}$-Acylrest ist.

9. Ester nach Anspruch 8, worin R Acetyl,Propionyl oder Acetoacetyl ist.